# EUROPEAN PATENT APPLICATION

(11) **EP 1 153 586 A2**
(43) Date of publication of application: **14.11.2001**
(21) Application number: 01111202.6
(22) Date of filing: 12.05.2001
(51) Int. Cl.: A61F 13/475, A61F 13/15

(54) **Sanitary napkin with breathable extension**

(30) Priority: 12.05.2000 US 570113; 09.04.2001 US 829391
(71) Applicant: JOHNSON & JOHNSON INC., Montreal, Quebec H1N 2G4 (CA)
(72) Inventor: Canuel, Louis, Quebec H6A 5V6 (CA)
(74) Representative: Groening, Hans Wilhelm, Dipl.-Ing.

(57) **Abstract**

A sanitary napkin including a liquid-absorbing section for absorbing body exudate and a breathable extension component adjacent the rear portion of the liquid-absorbing section. The breathable extension component is preferably free of absorbent material and has a moisture vapor transmission rate of at least about 3000 g/m²/day to allow moisture trapped between the breathable extension component and the skin of the wearer to evaporate.

## Description

### FIELD OF THE INVENTION

The present invention relates to an article for absorbing body exudate, more particularly to a sanitary napkin with a breathable extension component. In one embodiment, the breathable extension component is located in the rear portion of the sanitary napkin and is capable of reducing the likelihood of leakage, particularly at nighttime when the wearer has adopted a horizontal posture.

### BACKGROUND OF THE INVENTION

Traditional sanitary napkins, when worn during the night are prone to failures in the rear portion area, because the menstrual liquid has a tendency to flow under the effect of gravity toward the buttocks. This problem is less likely to happen during daytime when the wearer is standing up, as in such position, gravity actually causes the menstrual liquid to penetrate through the cover layer of the napkin and migrate toward the absorbent layer where it is permanently retained. To limit the occurrence of nighttime failures, manufacturers have developed specially designed napkins that offer enhanced absorptive capability near the rear portion. More specifically, such napkins are made longer and somewhat wider so as to offer a greater surface area to capture menstrual liquid. The theory behind this approach is that free liquid that may have traveled along the body of the wearer or along the cover layer, to the buttocks area of an article, that would not have been absorbed in an article designed for daytime use, would be absorbed in the rear extension of an article designed for nighttime use. Thus, the chances of failures are reduced.

Although sanitary napkins made for nighttime use are less likely to suffer from rear end failures in comparison with sanitary napkin designs made for daytime use, nighttime failures do still occur. One problem is that exudate to be absorbed may flow along the cover layer of the napkin in the gap created by the gluteal grove of the wearer. Conventional napkins do not address this problem as they have all been designed to lie relatively flat against the body. Furthermore, conventional napkins all have adhesive at the rear extremity of their garment-facing surface. This adhesive, amongst other things, will generally prevent the napkin from conforming to the shape of the buttocks (*e.g.* gluteal grove) in that region of the body.

Another drawback with conventional sanitary products for nighttime use is their less than ideal comfort potential. In this respect, for at least some consumers, the presence of a large rear extension creates a perception of a product that is excessively bulky, less comfortable, and impractical to install in an undergarment.

Furthermore, from a manufacturing point of view, conventional nighttime-use designs, although effective, are more expensive to produce than daytime-use designs because more raw materials are required to make the rear extension.

To address the drawbacks identified above, the industry has developed a sanitary napkin offering an enhanced protection against failures at the rear end, particularly when the wearer has adopted a horizontal posture. The sanitary napkin features an extension component that includes little or no absorptive material and that provides a liquid blocking function to limit the risks of failure. Another benefit of the extension component is the improvement in the stability of the sanitary napkin resulting from the larger surface area in contact with the body of the wearer. This sanitary napkin is described in a patent application in the name of the present assignee filed in the United States on November 9, 1998 under serial number 09/189,009. The contents of this application are incorporated herein by reference.

One potential difficulty with the sanitary napkin design featuring the rear extension component, or for that matter an extension component in any other part of the sanitary napkin is the sensation of wetness that such extension component can impart to the user. This is due primarily to the trapping of moisture between the extension component and the skin. The sensation of moisture is amplified when the cover layer of the sanitary napkin, forming one layer of the extension component is made of polymeric film that has a greater tendency to stick to the skin than covers made of fibrous materials.

### OBJECT AND STATEMENT OF THE INVENTION

It is therefore an object of the present invention to provide an improved sanitary napkin including a breathable extension component that is less likely to provide a sensation of wetness and a sticky feel when worn by comparison to known designs.

Hence, as embodied and broadly described herein, the present invention provides a sanitary napkin for placement in a crotch portion of an undergarment of a wearer, comprising:
a) a liquid-absorbing section for absorbing bodily exudate, said liquid-absorbing section including a longitudinal axis, a front portion and a rear portion, said liquid-absorbing section including:
   (i) a fluid-permeable cover layer;
   (ii) an absorbing system underneath said fluid-permeable cover layer;
   (iii) a liquid-impervious barrier layer underneath said absorbing system;
b) a breathable extension component projecting from said liquid-absorbing section, said breathable extension component including a proximal edge and a distal edge spaced from said proximal edge by a distance of at least about 2.5 cm, said proximal edge being united to said liquid-absorbing section;
c) said breathable extension component being adjacent the rear portion of said liquid-absorbing section;
d) said breathable extension component having at least a portion of its extent that manifests a moisture vapor transmission rate of at least about 3000 g/m²/day;
e) said breathable extension component having an average absorption capacity that is substantially less than an average absorption capacity of said liquid-absorbing section.

In a specific example of implementation, the liquid-absorbing section comprises at least three distinct layers united together in a superimposed relationship.

The layer that is normally in contact with the body of the wearer when the napkin is in use is conventionally termed the fluid-permeable cover layer or simply cover layer. The primary purpose of the cover layer is to permit the rapid ingress of exudate to be absorbed into the napkin, all the while remaining dry and comfortable to the user of the napkin.

The absorbent system is below the cover layer. The absorbent system may comprise a single layer or a plurality of layers. The primary purpose of this structure is to absorb and retain bodily exudate.

Underneath the absorbent system is the liquid-impervious barrier layer. The primary purpose of the barrier layer is to prevent exudate absorbed within the napkin from egressing the napkin on the opposite side from which it was absorbed.

Under this example of implementation, the breathable extension component projects from the rear portion of the liquid-absorbing section when the napkin is in a flat state. No particular shape is essential to the breathable extension component. Typically, the breathable extension component will be a natural extension of the liquid-absorbing section, *i*.*e*. it will be a continuation of the same overall shape. The breathable extension component extends from the rear of the liquid-absorbing portion at least 2.5 centimeters, preferably at least 3.75 centimeters, and more preferably at least 5.0 centimeters, and most preferably at least 7.0 centimeters. The length of extension of the breathable extension component is the distance between the proximal edge of the breathable extension component and the its distal edge. Furthermore, while not essential, it is preferred that the breathable extension component have a width of at least 7.0 cm, in order to cover a significant portion of the wearer's gluteal groove.

The breathable extension component may comprise a single layer of material or a composite laminate structure. In either case it is united with the liquid-absorbing section, preferably in one of two ways. In one execution, the breathable extension component is not continuous with any of the component layers of the liquid-absorbing section. Rather, the breathable extension component is manufactured separately from the liquid-absorbing section and later united therewith in such a manner so as to extend rearward from the liquid-absorbing section as a separate entity. In such cases the breathable extension component is joined with the liquid-absorbing section in an overlapping relationship.

By contrast, in the second and more preferred execution, the breathable extension component is continuous with, and is an integral extension of, one or more of the component layers of the liquid-absorbing section. The breathable extension component might simply be continuous with either the barrier layer of the liquid-absorbing section, the cover layer of the liquid-absorbing section, or preferably both.

In either embodiment, the breathable extension component will have two external surfaces, a first external surface facing the body of the wearer (specifically at least a portion of which will face the wearer's buttocks and gluteal groove) when the napkin is in use. It will also have a second external surface facing the wearer's undergarment. When the napkin is in use, the breathable extension component is positioned to lay flat against the skin of the wearer.

Preferably, the external surface of the breathable extension component that faces the undergarment of the wearer is substantially free of adhesive material. The absence of adhesive material will promote a better conformation of the breathable extension component to the buttocks of the wearer since the breathable extension component will not be maintained strongly against the wearer's undergarment. Because of natural moisture in this area of the body, it is likely that, in the absence of being adhered to the wearer's undergarment, the breathable extension component will naturally slightly adhere to the skin of the wearer, likely causing that extension to assume the contour of the body in that area thereby reducing the likelihood of exudate leakage from along the gluteal grove. In addition, adhesive is likely to hinder the breathability of the extension component and for that reason also it is not preferred to apply adhesive on the breathable extension component.

Most preferably, the breathable extension component of the sanitary napkin is devoid of any absorbent system. Thus, any absorption capacity that is present in the breathable extension component, is due to the small absorption capacity of the cover layer part and the thin void volume which may exist between the cover layer part and the barrier layer part that is capable of accepting and retaining liquid. In practice, however, the absorbent capacity of the breathable extension component is small by comparison of the absorption capacity of the liquid-absorbing section.

In general, it is highly preferred that the average absorption capacity of the breathable extension component be less than the average absorption capacity of the liquid-absorption section. The reduction of the absorbent capacity in the breathable extension component will typically translate into a reduction in manufacturing expense and will also enhance the breathability of the extension component by providing a path of less resistance for vapor to migrate through the layer(s) of the extension component. Ratios of 20:1, 26:1, and 29:1 (liquid-absorption section capacity to breathable extension component capacity) are increasingly preferred.

It is preferred that the flexibility of the breathable extension component along a transverse axis be greater than the flexibility of the liquid-absorbing section also measured along a transverse axis. In this manner, the breathable extension component will be able to more easily conform to the shape of the body of the wearer in the gluteal region. Ratios of the flexibility of the breathable extension component versus the flexibility of the liquid-absorbing section of 26:1, 38:1, and 43:1 are increasingly preferred.

It is further preferred that the average thickness of the breathable extension component be less than the average thickness of the liquid-absorbing section. The reduced thickness, along with the increased flexibility, will generally enhance the comfort of the breathable extension component as compared with the rear sections of prior art sanitary napkins designed for nighttime use. Ratios of 5:1, 8:1, and 11:1 (liquid-absorption section thickness to breathable extension component thickness) are increasingly preferred.

Moreover, the present inventor has also discovered that the breathable extension component behaves as a positioning system by enhancing the stability of the sanitary napkin against the perineal area of the user. As mentioned earlier, the breathable extension component adapts and follows the anatomy of the wearer in the buttocks area. Such adaptation results in a greater contact area of the sanitary napkin with the skin with the result that the napkin is less likely to shift in position, and to better maintain a fluid barrier.

As embodied and broadly described herein, the invention further includes a sanitary napkin for placement in a crotch portion of an undergarment of a wearer, comprising:
a) a liquid-absorbing section for absorbing bodily exudate, the liquid-absorbing section including a longitudinal axis, a front portion and a rear portion, two longitudinal sides opposed to one another and extending between said front portion and said rear portion, said liquid-absorbing section including:
   (i) a fluid-permeable cover layer;
   (ii) an absorbing system underneath said fluid-permeable cover layer;
   (iii) a liquid-impervious barrier layer underneath said absorbing system
b) a breathable extension component projecting from said liquid-absorbing section, said breathable extension component including a proximal edge and a distal edge spaced from said proximal edge by a distance of at least about 2.5 cm, said proximal edge being united to said liquid-absorbing section;
c) said breathable extension component including a segment projecting from a longitudinal side of said liquid-absorbing section, said segment being adjacent the rear portion of said liquid-absorbing section;
d) said segment manifesting a moisture vapor transmission rate of at least about 3000 g/m²/day;
e) said breathable extension component having an average absorption capacity that is substantially less than an average absorption capacity of said liquid-absorbing section.

As embodied and broadly described herein, the invention further includes a sanitary napkin for placement in a crotch portion of an undergarment of a wearer, comprising:
a) a liquid-absorbing section for absorbing bodily exudate, said liquid-absorbing section including:
   i) a longitudinal axis;
   ii) a front portion and a rear portion;
   iii) two longitudinal sides opposed to one another and extending between said front portion and said rear portion;
   iv) a pair of flaps projecting from said liquid absorbing section along generally opposite directions, each flap being adjacent to a respective longitudinal side and being capable of being folded about the crotch portion of the undergarment of the wearer when the sanitary napkin is placed in the crotch portion of the undergarment of the wearer;
   v) a fluid-permeable cover layer;
   vi) an absorbing system underneath said fluid-permeable cover layer;
   vii) a liquid-impervious barrier layer underneath said absorbing system;
b) a breathable extension component projecting from said liquid-absorbing section, said breathable extension component including a proximal edge and a distal edge spaced from said proximal edge by a distance of at least about 2.5 cm, said proximal edge being united to said liquid-absorbing section;
c) said breathable extension component including a portion that is spaced apart from one of said flaps relative the longitudinal axis of said liquid-absorbing section;
d) said portion manifesting a moisture vapor transmission rate of at least about 3000 g/m²/day;
e) said breathable extension component having an average absorption capacity that is substantially less than an average absorption capacity of said liquid-absorbing section.

Other objects and features of the invention will become apparent by reference to the following specification and to the drawings.

### BRIEF DESCRIPTION OF THE DRAWINGS

The following is a description by way of a preferred embodiment, reference being made to the following drawings, in which:
FIG. 1 is a top plan view of a sanitary napkin constructed in accordance with the present invention, showing the cover layer partially peeled off so as to reveal a transfer layer, an absorbent layer, and a barrier layer;
FIG. 2 is a perspective view of the sanitary napkin of the present invention in a curved conformation, typical of what would be attained when the napkin is placed in the undergarment of the wearer;
FIG. 3 is a bottom plan view of the sanitary napkin in Figure 1;
FIG. 4 is a sectional view taken along line 4-4 in Figure 1;
FIG. 5 is a side view of the sanitary napkin in accordance with the present invention shown secured to the undergarment of the wearer (the undergarment being illustrated in broken lines);
FIG. 6 is top plan view of another embodiment of the present invention in which the breathable extension component is discontinuous with the liquid-absorbing section;
FIG. 7 is a sectional view taken along line 7-7 in Figure 6;
FIG. 8 is a sectional view taken along line 8-8 in Figure 6;
FIG. 9 is a top plan view of a sanitary napkin in accordance with a variant featuring two breathable extension components that project laterally from the liquid-absorbing section; and
FIG. 10 is a schematical view of an apparatus for measuring the lateral flexibility of a sanitary napkin or components thereof.

In the drawings, preferred embodiments of the invention are illustrated by way of example. It is to be expressly understood that the description and drawings are only for purposes of illustration and as an aid to understanding, and are not indented to be a definition of the limits of the invention.

### DESCRIPTION OF A PREFERRED EMBODIMENT

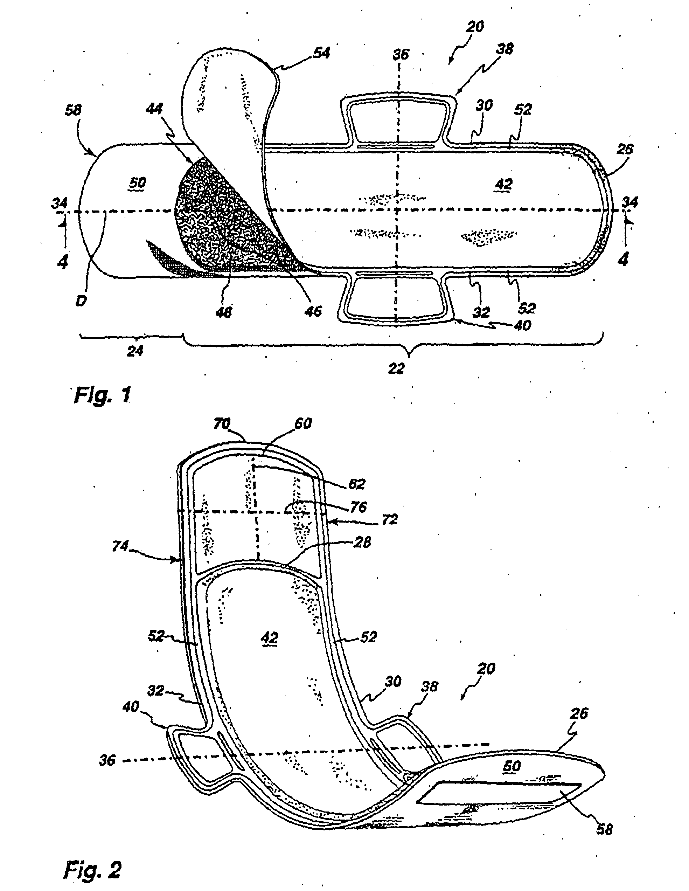

Referring to Figures 1 and 2, there is shown an embodiment of the present invention, a feminine sanitary napkin 20. The sanitary napkin 20 has a liquid-absorbing section 22 with a first transverse side 26 defining a front portion thereof and a second transverse side 28 defining a rear portion thereof. Each of these sides is arcuate, but may also be straight or any other suitable shape. The liquid-absorbing section 22 also has two longitudinal sides, namely a longitudinal side 30 and a longitudinal side 32. The liquid-absorbing section 22 has a thickness not exceeding about 5 mm. Preferably, the thickness is less than 3.5 mm, more preferably less than 3 mm, and most preferably, it is of about 2.8 mm.

A breathable extension component 24 projects from the second transverse side 28.

The sanitary napkin 20 has a longitudinal centerline 34 that is an imaginary line bisecting the sanitary napkin 20 in two identical halves.

The example of implementation of the invention depicted in Figure 1 of the drawings is a sanitary napkin with flaps. The flaps 38, 40 project laterally outward from each of the longitudinal sides 30, 32. The flaps 38, 40 are in the shape of an isosceles trapezoid with the top adjoining the longitudinal side and the base at the distal end. Other flap shapes can also be considered without departing from the spirit of the invention. Moreover, it should be expressly noted that flaps are not an essential component of the present invention, as a sanitary napkin without flaps and embodying the present inventive principle can be realised.

The liquid-absorbing section 22 also has an imaginary transverse centerline 36 perpendicular to the longitudinal centerline 34 and simultaneously bisecting the flaps 38,40.

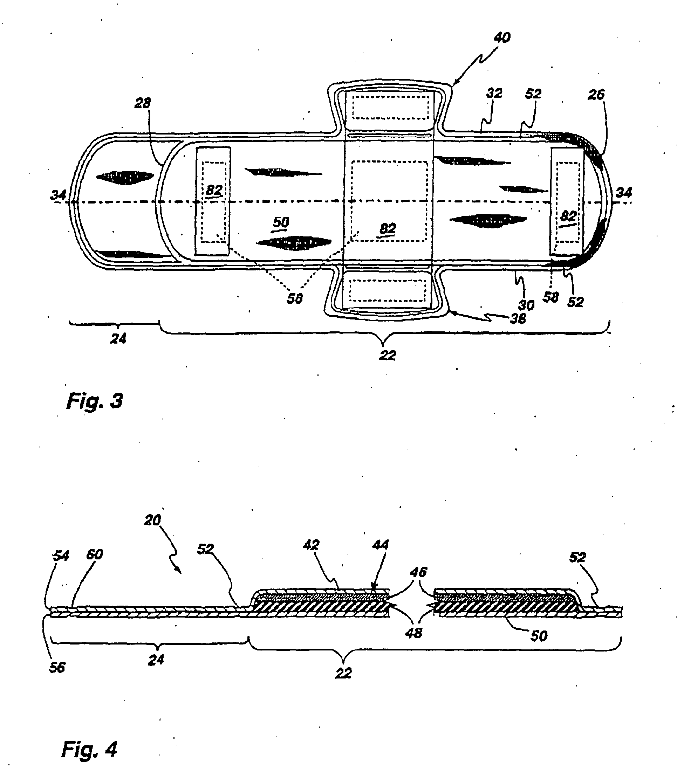

As depicted in Figure 4, the liquid-absorbing section 22 is of a laminate construction and preferably comprises a fluid-permeable cover layer 42, an absorbent system 44, and a fluid-impervious barrier layer 50. The absorbent system 44 has preferably two components, namely a first absorbent layer 46 (commonly known as "transfer layer") and a second absorbent layer 48 (commonly known as "absorbent core"). Alternatively, a single layer, namely the second absorbent layer 48, can form the absorbent system 44. Each of these layers is described in hereinbelow.

### Liquid-Absorbing Section - Cover Layer

The cover layer 42 may be a relatively low density, bulky, high-loft non-woven web material. The cover layer 42 may be composed of only one type of fiber, such as polyester or polypropylene or it may be composed of bi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof. An example is the non-woven cover layer of sanitary napkins sold by Johnson & Johnson Inc. of Montreal, Canada under the trademark Stayfree Ultra-Thin Cottony Dry Cover.

Bi-component fibers may be made up of a polyester core and a polyethylene sheath. The use of appropriate bi-component materials results in a fusible non-woven fabric. Examples of such fusible fabrics are described in U.S. Patent 4,555,446 issued November 26, 1985 to Mays. Using a fusible fabric increases the ease with which the cover layer may be mounted to the adjacent first absorbent layer and/or to the barrier layer.

The cover layer 42 preferably has a relatively high degree of wettability, although the individual fibers comprising the cover may not be particularly hydrophilic. The cover material should also contain a great number of relatively large pores. This is because the cover layer 42 is intended to take-up body fluid rapidly and transport it away from the body and the point of deposition. Advantageously, the fibers which make up the cover layer 42 should not lose their physical properties when they are wetted, in other words they should not collapse or lose their resiliency when subjected to water or body fluid. The cover layer 42 may be treated to allow fluid to pass through it readily. The cover layer 42 also functions to transfer the fluid quickly to the other layers of the absorbent system 44. Thus, the cover layer 42 is advantageously wettable, hydrophilic and porous. When composed of synthetic hydrophobic fibers such as polypropylene or bi-component fibers, the cover layer 42 may be treated with a surfactant to impart the desired degree of wettability.

Alternatively, the cover layer 42 can also be made of polymer film having large pores. Because of such high porosity, the film accomplishes the function of quickly transferring body fluid to the inner layers of the absorbent system. Apertured co-extruded films such as described in U.S. Patent 4,690,679 and available on sanitary napkins sold by Johnson & Johnson Inc. of Montreal, Canada could be useful as cover layers in the present invention.

The cover layer 42 may be embossed to the remainder of the absorbent system 44 in order to aid in promoting fluid transport by fusing the cover to the next layer. Such fusion may be effected locally, at a plurality of sites or over the entire contact surface of cover layer 42 with absorbent system 44. Alternatively, the cover layer 42 may be attached to the absorbent system 44 by other means such as by adhesion.

### Liquid-Absorbing Section - Absorbent System - First Absorbent Layer

Adjacent to the cover layer 42 on its inner side and bonded to the cover layer 42 is a first absorbent layer 46 that forms part of the absorbent system 44. The first absorbent layer 46 provides the means of receiving body fluid from the cover layer 42 and holding it until an underlying second absorbent layer has an opportunity to absorb the fluid.

The first absorbent layer 46 is, preferably, more dense than and has a larger proportion of smaller pores than the cover layer 42. These attributes allow the first absorbent layer 46 to contain body fluid and hold it away from the outer side of the cover layer 42, thereby preventing the fluid from re-wetting the cover layer 42 and its surface. However, the first absorbent layer 46 is, preferably, not so dense as to prevent the passage of the fluid through the layer 46 into the underlying second absorbent layer 48. These types of absorbent layers are commonly known as fluid transfer layers or acquisition layers.

The first absorbent layer 46 may be composed of fibrous materials, such as wood pulp, polyester, rayon, flexible foam, or the like, or combinations thereof. The first absorbent layer 46 may also comprise thermoplastic fibers for the purpose of stabilizing the layer and maintaining its structural integrity. The first absorbent layer 46 may be treated with surfactant on one or both sides in order to increase its wettability, although generally the first absorbent layer 46 is relatively hydrophilic and may not require treatment. The first absorbent layer 46 is preferably bonded on both sides to the adjacent layers, i.e. the cover layer 42 and an underlying second absorbent layer 48. An example of a suitable first absorbent layer is a through air bonded pulp sold by BUCKEYE of Memphis Tennessee under the designation VIZORB 3008.

### Liquid-Absorbing Section ― Absorbent System ― Second Absorbent Layer

Immediately adjacent to and bonded to the first absorbent layer 46 is the second absorbent layer 48.

In one embodiment, the first absorbent layer 46 has a central width that is at least about the same as the central width of the second absorbent layer 48. In a specific embodiment, this central width is greater than about 64mm. In another embodiment, the first absorbent layer 46 has a central width that exceeds the central width of the second absorbent layer 48. The term "central width" refers to a specific area of a layer, such as an absorbent layer determinable as follows. A reference point on the sample layer that is disposed beneath the center of the vaginal orifice, when worn, is located. A plane parallel to the transverse centerline 36 and 3.75 centimeters forward from the reference point in the direction of the wearer's mons pubis is located. Another plane parallel to the lateral centerline 36 and 5.0 centimeters rearward from the reference point in the direction of the wearer's buttocks is also located. The greatest flat-out, uncompressed, unmanipulated, lateral width of the sample layer between the two planes is the absorbent width of the sample layer.

The central width of the absorbent system, when the absorbent system includes a plurality of absorbent layers is the central width of the layer of the absorbent system that has the largest central width. In a specific example, the central width of the absorbent system exceeds 64 mm.

In one embodiment, the second absorbent layer 48 is a blend or mixture of cellulosic fibers and superabsorbent disposed in and amongst the cellulosic fibers.

Details on the structure and the method of construction of the second absorbent layer 48 are available in US patent 5,866,242 granted on February 2, 1999 to Tan et al. The contents of this document are hereby incorporated by reference.

### Liquid-Absorbing Section - Barrier Layer

Underlying the absorbent system 44 is a barrier layer 50 comprising liquid-impervious material so as to prevent liquid that is entrapped in the absorbent system 44 from egressing the sanitary napkin and staining the wearer's undergarment. The barrier layer 50 is preferably made of polymeric film. Most preferably, the barrier layer 50 is made of polymeric film that is breathable such as that, for example, available by Tredegar Corporation of Illinois under the designation 116 MPF. A breathable film is capable of blocking the passage of liquid while permitting gas or vapor to pass through. Alternatively, the barrier layer may be made of other than film material, such as woven or non-woven material suitably treated to prevent the passage of liquid through the material.

Several suggestions exist for rendering a relatively liquid impervious polyethylene film, pervious to water vapor transmission. For example, in U.S. Pat. No. 3,989,867 issued to J. B. Sisson on Nov. 2, 1976 a polyethylene barrier layer is described having cone shaped bosses which will allow water vapor to evaporate from the surface of the absorbent material in a diaper, sanitary napkin or dressing. The actual open surface of such a barrier layer is said to be from about 1/2% to about 10%.

Another possibility is to use a barrier layer developed for use in the surgical dressings and described in U.S. Pat. No. 3,426,754 issued on Feb. 11, 1969 to Harvey S. Bierenbaum, et al. Described therein is a polymeric film having what is termed micropores i.e., extremely small openings.

Yet another possibility is to use as a barrier layer a fibrous, vapor permeable, liquid repellent layer of the type described in the U.S. Patent No. 4,698,876 to Becker and al.

The cover layer 42 and the barrier layer 50 are joined along their marginal portions so as to form an enclosure or flange seal that maintains the absorbent system 44 captive. The joint may be made by means of adhesives, heat-bonding, ultrasonic bonding, radio frequency sealing, mechanical crimping, and the like and combinations thereof. The peripheral seal line is shown in Figure 1 by the reference numeral 52.

### Adhesive System

Referring to Figures 2 and 3, in order to enhance the stability of the sanitary napkin, the garment facing surface of the barrier layer is provided with positioning adhesive material 82, typically hot-melt adhesive material capable of establishing a temporary bond with the undergarment material. A suitable material is the composition designated HL-1491 XZP commercially available from H.B. Fuller Canada, Toronto, Ontario, Canada.

The preferred form of construction of the invention also includes positioning adhesive zones on the flaps 38 and 40.

Standard release paper 58 (shown only in Figures 2 and 3) covers the positioning adhesive pattern before the napkin is used to prevent the unwanted adherence of the napkin to itself or foreign objects. The release paper is of conventional construction (e.g. silicone coated wet-laid Kraft wood pulp) and suitable papers are available from Tekkote Corporation (Leonia, New Jersey, USA), and bear the designation FRASER 30#/61629.

### The Breathable Extension Component

United with the liquid-absorbing section 22 is the breathable extension component 24. The breathable extension component 24 is a thin and flexible extension adjacent the rear portion of the liquid-absorbing section 22, capable of conforming to the buttocks area of the wearer. The breathable extension component 24 appears as a natural extension of the liquid-absorbing section 22, and thus its longitudinal sides generally appear as extensions of the longitudinal sides 30, 32 of the liquid-absorbing section 22.

The breathable extension component 24 projects from the rear of the liquid-absorbing section 22 approximately 7.0 cm (*i*.*e*. its projection length 62 is approximately 7.0 cm). The projection length 62 of the breathable extension component 24 is the maximum distance that exists between the distal side 70 of the breathable extension component 24 and the proximal side 28 of the breathable extension component 24 (including the width of the seal 60, if any), measured along a line that is parallel to the longitudinal centerline 34 of the liquid-absorbing section 22. The breathable extension component 24 also has a width 76 of approximately 7.0 cm; the width 76 of the section 24 being the distance from one longitudinal side 72 of the section 24 to the other 74, measured along a line perpendicular to the line along which the projection length 62 is measured, through the midpoint thereof.

Referring to Figure 4, the breathable extension component 24 is a dual-layer structure comprising a cover layer part 54 that is continuous (and integrally-formed) with the cover layer 42 of the liquid-absorbing section 22. Both the cover layer part 54 and the cover layer 42 are formed of the same material. Optionally, the cover layer part 54 of the breathable extension component 24 may contain a non-woven fabric to reduce what some users might perceive to be a hot sticky plastic feel; or it may be preferred to use a combination of a plastic cover with non-woven material in the breathable extension component 24. The breathable extension component 24 also comprises a barrier layer part 56 that is continuous (and integrally-formed) with the barrier layer 50 of the liquid-absorbing section 22. Both the barrier layer part 56 and the barrier layer 50 are formed of the same material. The cover layer part 54 is secured to the barrier layer part 56 along a generally U-shaped peripheral seal line 60. Thus the peripheral seal 52 in the second transverse end region of the liquid-absorbing section and the seal line 60 define the breathable extension component 24 in the present embodiment. Since the barrier layer part 56 is breathable and the cover layer part 54 is inherently breathable, the breathable extension component 24 is breathable and thus permits moisture or vapor to pass through. This allows eliminating moisture that may accumulate on the skin of the wearer, making the sanitary napkin more comfortable to wear.

No portion of the absorbent system 44 extends into the breathable extension component 24, nor are there any additional independent absorbent materials present therein (except for the perhaps minimally absorbent cover layer part 54). The breathable extension component 24 is thus of reduced absorbent capacity and reduced thickness as compared with the liquid-absorbing section 22. The difference in thickness between the liquid-absorbing section 22 and the breathable extension component 24 that results from the absence of absorbent material in the breathable extension component 24 is clearly depicted in Figure 4. The ratio of average absorption capacity of the liquid-absorbing section 22 to average absorption capacity of the breathable extension component 24 is at least 20:1. Similarly, the ratio of thickness of the liquid-absorbing section 22 to the thickness of the breathable extension component 24 is at least 5:1. The ratio of the flexibility of the liquid-absorbing section 22 to the flexibility of the breathable extension component 24 is at least 1:26. Exact values for each of these characteristics and ratios may be determined using the techniques described in the section entitled "Test Procedures".

The breathable extension component 24 has a moisture vapor transmission rate of at least about 3000 g/m²/day, and preferably of at least about 4000 g/m²/day, and more preferably of at least about 5000 g/m²/day. In one possible form of implementation, the barrier layer 50 is breathable over its entire extent and as such it donates or at least contributes to the breathability of the breathable extension component 24. Alternatively, the barrier layer 50 is non-breathable and only the breathable extension component 24 is breathable. The example of implementation shown in Figure 4 has a breathable extension component 24 made of two parts, namely a cover layer part 54 that is continuous (and integrally-formed) with the cover layer 42 and a barrier layer part 56 that is continuous (and integrally-formed) with the barrier layer 50. In a situation where one desires to obtain breathability only in the area of the breathable extension component 24, this can be achieved by suitably treating the barrier layer part 56 to make it breathable, while keeping the rest of the barrier layer 50 non-breathable.

Referring to Figure 3, the garment-facing surface of barrier layer part 56 of the breathable extension component 24 does not contain positioning adhesive material 58 as does the garment-facing surface of the barrier layer 50 of the liquid-absorbing section 22. The breathable extension component 24 is therefore not adhered to the undergarment when the napkin 20 is in use and is free to lift off and conform to the body of the wearer. Although the breathable extension components of the embodiments described in the figures do not contain positioning adhesive material on their garment-facing surfaces, it should be expressly understood that the application of positioning adhesive material on the garment-facing surfaces of the breathable extension components, however, remains within the scope of this invention. Small amounts of adhesive are not likely to have a major negative impact on the overall breathability of the breathable extension component. The results of tests that have been conducted in order to assess the impact of positioning adhesive material on the MVTR capability of a sample have revealed that a positioning adhesive material disposed as a plurality of spaced-apart parallel lines, for example, has less impact on the MVTR of an article than does positioning adhesive material that is disposed as a continuous block.

### Method of Manufacture

The above-described embodiment of the sanitary napkin 20 is fabricated in a conventional manner in accordance with conventional techniques. Specifically, a laminate structure, sometimes referred to in the art as a web, is created. This laminate structure comprises an expanse of the materials from which the napkin will be created. I.e. the laminate structure comprises the following layers of material in a top-to-bottom order: an expanse of cover layer material; an expanse of first absorbent layer material; an expanse of second absorbent layer material; and finally an expanse of barrier layer material. Some of the materials are necessarily not continuous within the laminate structure, and where such is the case, they are positioned precisely, one with respect to another, in the relationship they will occupy in the final products. The cover layer material and the barrier layer material are then bonded together by applying pressure in the appropriate positions, and what will become the peripheral seal is created. The seal may also be made by means of heat-bonding, ultrasonic bonding, radio frequency sealing, mechanical crimping, and the like and combinations thereof. The sealed structure is then severed by conventional means (i.e. die-cutting, fluid-jet cutting, or by laser) from the web to create a discrete article.

The positioning adhesive material is then applied to the barrier layer to create the longitudinal adhesive zones 82, and any other adhesive zones as the case may be, and release paper is applied to cover the positioning adhesive. Alternatively, the positioning adhesive, or the positioning adhesive and the release paper may be applied to the web before the individual articles are severed therefrom.

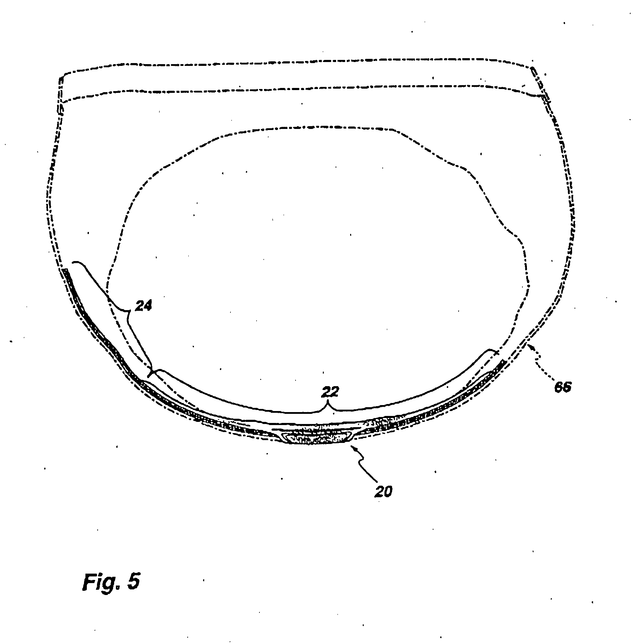

Figure 5 illustrates the sanitary napkin 20, positioned in an undergarment 66 shown in broken lines. The sanitary napkin 20 is depicted in the position it would attain when the undergarment 66 is in use, pulled such that its crotch portion presses against the perineal area of the user. In this position the liquid-absorbing section 22 of the sanitary napkin 20 covers at least in part the vaginal opening of the user. The breathable extension component 24 of the sanitary napkin 20 extends over at least a portion of the buttocks area. As mentioned earlier in this specification, the buttocks area is defined as the seat portion of the body that extends upwardly from the anal opening up to the lower rear portion. The breathable extension component 24 will conform to the shape of the body of the wearer in the gluteal region, and will thus act to prevent rear end failures, particularly when the wearer is in a horizontal prone position. The breathable extension component 24 will also help to position the napkin as described above.

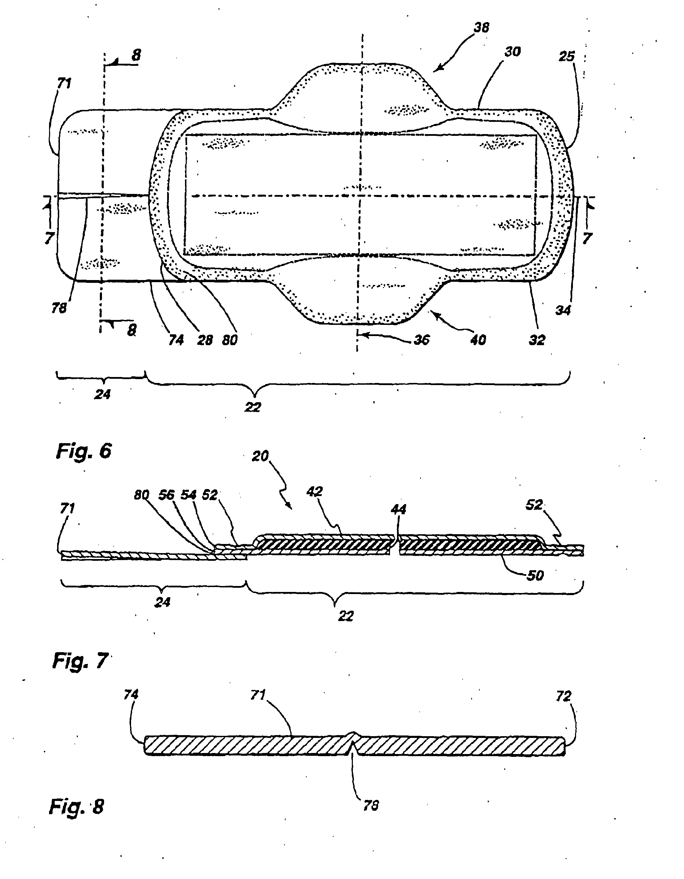

Another embodiment of the present invention is shown in Figure 6. In this embodiment the liquid-absorbing section 22 and the breathable extension component 24 are discrete components with a physical line of demarcation 80 between them. The liquid-absorbing section 22 in this embodiment is generally similar to that described above in association with other embodiments, and is manufactured in a similar fashion. Referring to Figure 7, the breathable extension component 24, comprises a single layer of the breathable and liquid impervious material 71. The single layer of breathable and liquid impervious material 71 may comprise the same material as the barrier layer 50 or different material from the barrier layer 50. The breathable extension component 24 is independently cut from a web of material and is united by adhesive or other conventional means to the liquid-absorbing section 22 after that section's manufacture. The breathable extension component 24 contains a preferential bending zone in the form of a score line 78. The score line 78 is co-linear with the longitudinal centerline 34 of the liquid-absorbing section 22. Referring to Figure 8, the score line 78 is such that when the napkin is in use the breathable extension component 24 will preferentially bend inwards into the wearer's gluteal grove. This preferential bending will augment the ability of the breathable extension component 24 to act as a positioning system, as described above.

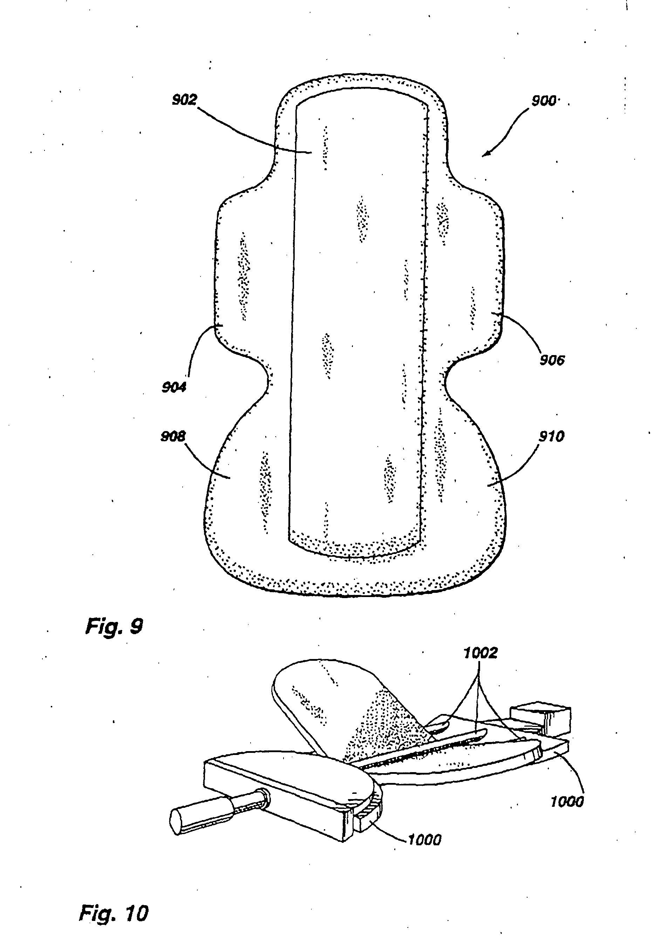

Figure 9 illustrates a variant of the sanitary napkin in accordance with the invention. The sanitary napkin 900 includes a liquid-absorbing section 902 from which project laterally a pair of flaps 904 and 906 that fold about the edges of the undergarment when the sanitary napkin 900 is in use. The sanitary napkin 900 also includes a breathable extension component including a pair of lateral segments 908 and 910 adjacent the rear portion of the liquid-absorbing section 902 and spaced form the respective flaps 904 and 906 relative the longitudinal axis of the sanitary napkin. The lateral segments 908 and 910 project outwardly from the respective side edges of the liquid-absorbing section 902 and are made from co-extensive projecting portions of the cover layer and the barrier layer of the sanitary napkin, without any absorbent element between them These co-extensive portions are united to one another in any suitable manner. This structure allows moisture between the skin of the wearer and the segments 908 and 910 to evaporate through those segments. Note that in this example, the entire barrier layer of the sanitary napkin could be breathable. Another possibility is to treat only the portions of the barrier layer that extend in the lateral segments 908 and 910 to make them breathable and keep the remainder of the barrier layer non-breathable.

### TEST PROCEDURES

### Thickness

The apparatus required to measure the thickness of the sanitary napkin is a footed dial (thickness) gauge, available from Ames, with foot 1 1/8" diameter with stand, 2-oz. deadweight accurate to 0.001". A digital type apparatus is preferred. If the sanitary napkin sample is individually folded and wrapped, the sample is unwrapped and carefully flattened by hand. The release paper is removed from the sample and it is repositioned back gently across the positioning adhesive lines so as not to compress the sample, ensuring that the release paper lies flat across the sample. Flaps (if any) are folded back under the sample, prior to taking the thickness reading in the center of the sample.

The foot of the gauge is raised and the sample is placed on the anvil such that the foot of the gauge is approximately centered to the sample (or in the location of interest on the sample of interest). When lowering the foot, care is taken to avoid allowing the foot to "drop" or that undue force is not applied. The read out is allowed to stabilize for approximately 5 seconds. The thickness reading is then taken.

### Lateral Flexibility/Stability

The purpose of this test is to determine the lateral flexibility of an absorbent product or a portion of an absorbent product by subjecting the sample to side compression. The test procedure will be described with reference to Figure 10. The lateral flexibility of the sample is obtained by placing the sample in between two Plexiglas curves 1000 (simulating the inner thighs of the wearer), there being a stationary curve and a movable one. The stationary curve 1000 is attached to a compression load cell in an Instron Unit (not shown) and a speed of 500 millimeters per minute is imparted to the moving curve. The sample is securely placed in between the two curves 1000 with three metal support rods 1002 stemming from the stationary curve 1000 and slidingly received in the moving curve 1000. The force required to compress the sample to one-inch (2.54-cm) is recorded. In the case of the embodiment shown at Figure 4, the lateral flexibility is obtained by cutting each sample along the boundary separating the liquid-absorbing section 22 and the breathable extension component 24. Each sample is subjected to the testing procedure and the respective flexibility value is recorded. Note that each sample is placed in the instrument such that its longitudinal axis is perpendicular to the direction of movement of the moving curve.

### Absorption Capacity

The average absorption capacity can be determined by using any standard test procedure (i.e., the test using the "GATTS" measuring instrument, the Dunk test, etc.). In the Dunk test, a pre-cut sample is immersed into a container of 0.9% Saline Solution for a time of 10 minutes and then removed in order to be left to drip at an angle of 90° for 2 minutes. The average absorption capacity is obtained by subtracting the dry weight from the wet weight and is expressed, for both the liquid-absorbing section and the breathable extension component, in terms of weight per unit surface area.

### Moisture Vapor Transmission rate (MVTR)

A method that can be used to measure the moisture vapor transmission rate (MVTR) of an absorbent product or a portion thereof utilises a Payne permeability cup. In this method, a precise volume of 10 ml of de-ionized water is introduced into the Payne cup; the latter being a metal cup featuring a flange which delineates an exposed surface area of 10 cm². A pre-cut sample of an absorbent product is then placed over the flange of the cup and clamped over the latter. When the test sample is a sanitary napkin, it is placed over the flange of the cup with its barrier side facing upwardly. The cup is subsequently weighed and placed within a preconditioned chamber that is maintained at 37° +/- 1°C and 10% relative humidity. The cup and sample are then removed from the preconditioned chamber after a time of exposure of 24 hours and are allowed to cool to room temperature for 20 minutes prior to being re-weighed. The MVTR of the sample, which is expressed in g/m²/day, can then be calculated by determining the difference between the initial and final weights of the cup and multiplying that value by 1000.

## Claims

1. A sanitary napkin for placement in a crotch portion of an undergarment of a wearer, comprising:
a) a liquid-absorbing section for absorbing bodily exudate, said liquid-absorbing section including a longitudinal axis, a front portion and a rear portion, said liquid-absorbing section including:
(i) a fluid-permeable cover layer;
(ii) an absorbing system underneath said fluid-permeable cover layer;
(iii) a liquid-impervious barrier layer underneath said absorbing system
b) a breathable extension component projecting from said liquid-absorbing section, said breathable extension component including a proximal edge and a distal edge spaced from said proximal edge by a distance of at least about 2.5 cm, said proximal edge being united to said liquid-absorbing section;
c) said breathable extension component being adjacent the rear portion of said liquid-absorbing section;
d) said breathable extension component having at least a portion of its extent that manifests a moisture vapor transmission rate of at least about 3000 g/m²/day;
e) said breathable extension component having an average absorption capacity that is substantially less than an average absorption capacity of said liquid-absorbing section.

2. A sanitary napkin as recited in claim 1, wherein said breathable extension component has a moisture vapor transmission rate of at least about 4000 g/m²/day.

3. A sanitary napkin as recited in claim 1, wherein said breathable extension component has a moisture vapor transmission rate of at least about 5000 g/m²/day.

4. A sanitary napkin as recited in claim 1, wherein said breathable extension component has a longitudinally extending preferential bending zone, such that when the article is in use by the wearer at least a portion of said breathable extension component is capable of bending at said preferential bending zone so as to enter the wearer's gluteal groove.

5. A sanitary napkin as recited in claim 1, wherein said liquid-absorbing section and said breathable extension component each have an average absorption capacity, the average absorption capacity of said breathable extension component being less than the average absorption capacity of said liquid-absorbing section.

6. A sanitary napkin as recited in claim 5, wherein the average absorption capacity of said liquid-absorbing section and said breathable extension component have a ratio of about 20:1.

7. A sanitary napkin as recited in claim 1, wherein the liquid-absorbing section and said breathable extension component each have a thickness, the thickness of said breathable extension component being less than the thickness of said liquid-absorbing section.

8. A sanitary napkin as recited in claim 7, wherein the thickness of said liquid-absorbing section and said breathable extension component have a ratio of about 5:1.

9. A sanitary napkin as recited in claim 1, wherein said liquid-absorbing section and said breathable extension component each have a lateral flexibility, the lateral flexibility of said breathable extension component being greater than the lateral flexibility of said liquid-absorbing section.

10. A sanitary napkin for placement in a crotch portion of an undergarment of a wearer, comprising:
a) a liquid-absorbing section for absorbing bodily exudate, said liquid-absorbing section including a longitudinal axis, a front portion and a rear portion, two longitudinal sides opposed to one another and extending between said front portion and said rear portion, said liquid-absorbing section including:
(i) a fluid-permeable cover layer;
(ii) an absorbing system underneath said fluid-permeable cover layer;
(iii) a liquid-impervious barrier layer underneath said absorbing system
b) a breathable extension component projecting from said liquid-absorbing section, said breathable extension component including a proximal edge and a distal edge spaced from said proximal edge by a distance of at least about 2,5 cm, said proximal edge being united to said liquid-absorbing section;
c) said breathable extension component including a segment projecting from a longitudinal side of said liquid-absorbing section, said segment being adjacent the rear portion of said liquid-absorbing section;
d) said segment manifesting a moisture vapor transmission rate of at least about 3000 g/m²/day;
e) said breathable extension component having an average absorption capacity that is substantially less than an average absorption capacity of said liquid-absorbing section.
